# FASCICULE DE BREVET EUROPEEN

(11) **EP 0 702 565 B1**
(45) Date de publication et mention de la délivrance du brevet: **08.12.1999**
(21) Numéro de dépôt: 95914428.8
(22) Date de dépôt: 27.03.1995
(51) Int. Cl.: A61K 39/106, C12N 9/08

(54) **Composition immunisante à base de catalase d'Helicobacter pylori**
Auf Helicobacter pylori Katalase basierende immunisierende Zusammensetzung
Helicobacter pylori catalase-based immunising composition

(30) Priorité: 08.04.1994 FR 9404172
(43) Date de publication de la demande: 27.03.1996
(73) Titulaire: PASTEUR MERIEUX SERUMS ET VACCINS, 69007 Lyon (FR)
(72) Inventeur: LISSOLO, Ling, F-69280 Marcy l'Etoile (FR)
(74) Mandataire: Bernasconi, Jean
(86) Numéro de dépôt international: FR9500383
(87) Numéro de publication internationale: WO9527506

(56) Documents cités:
- EP-A- 0 367 644
- JOURNAL OF GENERAL MICROBIOLOGY, vol. 137, no. PART1, Janvier 1991 COLCHESTER,GB, pages 57-61, S.L. HAZELL ET AL. 'HELICOBACTER PYLORI CATALASE.' cité dans la demande
- EUROPEAN JOURNAL OF CLINICAL MICROBIOLOGY AND INFECTIOUS DISEASES, vol. 11, no. 6, Juin 1992 WIESBADEN,DE, pages 522-526, T.U. WESTBLOM 'CATALASE NEGATIVE MUTANTS OF HELICOBACTER PYLORI.' cité dans la demande

## Description

La présente invention a pour objet une composition pharmaceutique destinée au traitement ou à la prévention des infections à *Helicobacter pylori*.

*H. pylori* est une bactérie à gram négatif retrouvée exclusivement à ce jour à la surface de la muqueuse de l'estomac chez l'homme, et plus particulièrement autour des lésions de cratères des ulcères gastriques et duodénaux. Cette bactérie était initialement appelée *Campylobacter pyloridis* (Warren & Marshall (1983) Lancet 1 : 1273-1275).

*H. pylori* est à l'heure actuelle reconnu comme l'agent étiologique des gastrites antrales, et apparaît comme un des cofacteurs requis pour le développement des ulcères. Par ailleurs il semble que le développement des carcinomes gastriques puisse être associé à la présence de *H. pylori*.

Divers facteurs contribuent à l'infection. Cette bactérie est en particulier dotée d'une forte activité uréasique conférée par une uréase qui lui permet de survivre dans l'environnement acide de l'estomac. D'autre par, les flagelles lui confèrent une mobilité lui permettant de franchir le mucus afin de gagner sa niche écologique et d'adhérer à la muqueuse via des adhésines.

En dépit d'une importante réponse immune sérique et d'une réponse inflammatoire très prononcée, l'infection bactérienne est persistante. Une hypothèse très vraisemblable donnerait à une catalase produite par l'agent pathogène, un rôle central dans la chronicité de l'infection. En effet, les catalases (EC 1.11.1.6) sont en général des enzymes importantes pour la survie des bactéries en présence de H₂O₂. Ainsi, dans le cas présent, une catalase permettrait à *H. pylori* d'échapper aux effets toxiques des radicaux d'oxygène produits lors du phénomène inflammatoire.

Une catalase a déjà été caractérisée chez *H. pylori* (Hazell et al, J. Gen. Microbiol. (1991) 137 : 57). Il s'agit d'un tétramère dont la sous-unité a un poids moléculaire d'environ 50-60 kD. La séquence N-terminale d'une telle catalase a aussi été établie (Westblom et al, Eur. J. Clin. Microbiol. Inf. Dis. (1992) 11 (6) : 522). Cependant, il n'est pas exclue que cette bactérie possède plusieurs catalases, légèrement différentes entre elles. EP 367 644 décrit l'utilisation d'une protéine à activité d'uréase dans une composition immunogène pour la prévention de l'infection d'un individu par C. pylori.

On a maintenant trouvé qu'une catalase d'*H*. *pylori* pouvait être utilisée en tant que substance immunisante à l'encontre de l'agent pathogène lui-même, et pouvait être notamment utile à des fins prophylactiques ou thérapeutiques.

C'est pourquoi l'invention a pour objet une composition immunisante à l'encontre d'*H*. *pylori* qui comprend à titre de principe actif, une catalase d'*H. pylori* sous forme purifiée.

Une catalase utile aux fins de la présente invention peut être soit obtenue par extraction à partir d'*H. pylori* ou être obtenue par voie recombinante, par expression du fragment d'ADN correspondant, dans un système hétérologue, bactéries, levures ou cellules de mammifères.

A cet effet, on indique qu'une catalase peut être purifiée à partir d'*H. pylori* selon une technique identique ou similaire à celle décrite par Hazell et al, J. of Gen. Microbiol. (1991à 137 : 57. De même, on note qu'un fragment d'ADN codant pour cette protéine a été cloné et séquencé par Newell et al, Microbiol. Ecology in Health and Disease (1991) 4 : Supplement S120, H3-1.

Une catalase utile aux fins de la présente invention peut avoir conservé son activité catalasique ou bien l'avoir perdue par mutation, de préférence ponctuelle.

Une catalase utile aux fins de la présente invention peut être encapsulée dans un vecteur de délivrance tel que les liposomes ; ceci est particulièrement avantageux lorsque la catalase est destinée à une administration par voie orale.

Une composition selon l'invention peut en outre contenir un antigène d'*H. pylori* autre qu'une catalase ; par exemple une uréase.

Une composition selon l'invention peut être fabriquée de manière conventionnelle. En particulier on associe la catalase avec un adjuvant, un diluant ou un support acceptable d'un point de vue pharmaceutique. Une composition selon l'invention peut être administrée par n'importe quelle voie conventionnelle en usage dans le domaine des vaccins, en particulier par voie orale ou parentérale. L'administration peut avoir lieu en dose unique ou répétée une ou plusieurs fois après un certain délai d'intervalle. Le dosage approprié varie en fonction de divers paramètres, par exemple, de l'individu traité ou du mode d'administration.

### Exemple 1 : Purification d'une catalase à partir d'H. pylori

◆ Culture :
   A partir d'une souche d'*H. pylori* n° ATCC 43579 (disponible auprès de l'ATCC, 12301 Parklawn drive, Rockville MD - U.S.A.) conservée en glycérol à -70°C, on ensemence un flacon de 25 cm² contenant un milieu biphasique. Le milieu biphasique comprend une phase solide constituée de 10 ml de Trypticase soja Agar (Difco) additionné de 6 % de sang de mouton frais et une phase liquide constituée de 3 ml de bouillon Trypticase soja (Difco) contenant 20 % de sérum de veau foetal. Les flacons sont placés dans un sac étanche dit "generbag" (BBL) et incubés sous agitation rotative douce à 37°C pendant 48 heures en condition de microaérophilie (8-10 % CO₂, 5-7 % O₂ et 85-87 % N2) obtenu par le System Microaer® (BBL). Après 48 heures de cultures, une sous-culture est réalisée dans les mêmes conditions et ce, afin d'augmenter la biomasse. Les germes sont récoltés par centrifugation et lavés par du PBS (Biomérieux) afin d'éliminer le milieu de culture résiduel.
◆ Purification :
   Le culot bactérien lavé est remis en suspension dans du tampon phosphate de sodium 50 mM pH 7,5 contenant du PMSF (phenylmethylsulfonyl fluoride Sigma) 100 µM (tampon A) à concentration finale de 0,1 g (poids humide) par millilitre. La suspension est homogénéisée à l'aide d'un mixeur de type Ultraturrax®. Les cellules bactériennes sont ensuite cassées par sonication avec un appareil de type Sonifier® (Branson) équipé d'une sonde de diamètre 1,8 cm. La sonication s'effectue par intermittence, 1 min de sonication et 1 min de repos sur glace. Une sonication de 10 min est suffisante pour casser complètement 5 g de germes en suspension. Le lysat ainsi obtenu est centrifugé pendant 15 min à 4°C à 4 000 g. Le surnageant est récupéré puis, centrifugé à nouveau à 100 000 g pendant 30 min à 4°C. Le surnageant de cette deuxième centrifugation (S2) est récupéré pour une purification chromatographie. La fraction S2 préparée de cette façon conserve environ 90% de l'activité enzymatique "catalase" totale, telle que mesurée selon la technique de Hazell et al (supra) ou Beers & Sizer, J. Biol. Chem. (1952) 195 : 133.
   La fraction S2 est chargée sur une colonne S-Sépharose® (Pharmacia) préalablement équilibrée avec du tampon A. La colonne est lavée avec le même tampon. La chromatographie est suivie avec un détecteur UV à 280 nm pour les protéines et par l'activité enzymatique pour la catalase. Après élimination des protéines non-fixées (l'absorption à 280 nm revient à la ligne de base), la colonne est ensuite lavée avec un gradient de NaCl, 0 à 1M dans du tampon A. Les fractions correspondant au pic de l'activité catalase sont recueillies, concentrées dans une cellule de concentration de type Amicon® équipée de membrane dont le seuil de coupure est de 100 000 Daltons. La fraction concentrée ainsi obtenue est chargé sur une colonne Séphacryl® S-300 HR préalablement équilibrée avec du tampon PBS. Les fractions contenant de l'activité catalase sont recueillies, concentrées a 1 mg/ml et dialysées contre le tampon PBS. La solution finale est filtrée sur une membrane de porosité 0,22 µm et conservée à -70 °C.
   La protéine ainsi purifiée présente les caractéristiques suivantes:
   (1) Une activité enzymatique de catalase typique, en l'absence d'activité peroxydase.
   (2) Un spectre visible typique d'une hémoprotéine, un pic de Soret à 406 nm et les pics d'alpha et de bêta entre 520 nm et 550 nm.
   (3) Une forme monomerique à 54 kD en SDS-PAGE avec la séquence N-terminale suivante:
      MVNKDVKQTTAFGAPVWDDNNVITAGPRG

### Exemple 2 : Composition pharmaceutique destinée à une administration orale

La protéine obtenue dans l'exemple 1 peut être encapsulée seule ou en présence d'autres protéines de *H. pylori* dans des capsules de gélatine afin de protéger l'antigène contre la dégradation par le suc gastrique ou bien administrée en présence de bicarbonate de sodium. De telles formulations ont déjà été utilisées pour des compositions pharmaceutiques (Black et al, Dev. Biol. Stand.(1983) 53). La protéine peut également être encapsulée dans des microsphères de PLGA (copolymères d'acide glycolique et acide lactique) selon le protocole décrit ailleurs (Eldridge et al, Curr. Top. Microbiol. Immunol. (1989) 146 : 59-66). la protéine peut également être incluse dans des liposomes préparés selon les méthodes conventionnelles largement décrites ("Liposomes: a practical approach, ed. RRC New, D. Rickwood & B:D. Hames, 1990, Oxford University Press, ISBN 0-19-963077-1).

Indépendamment de la formulation, la quantité de protéine administrée à l'homme par voie orale est de l'ordre de 1 à 10 mg par prise, et l'on préconise au moins 3 prises à intervalle de 4 semaines.

### Exemple 3 : Composition pharmaceutique destinée à une administration parentérale

La protéine comme dans l'exemple 1 est adsorbée sur gel d'alumine de façon tout à fait conventionnelle. La protéine en solution à 1 mg/ml dans un tampon dont le pH est voisin de 6.5 est mise en contact pendant 1 heure avec de l'hydroxyde d'aluminium à 10 mg/ml mesuré en Al⁺⁺⁺. La composition finale de la préparation est la suivante : protéine à activité catalasique 50 µg/ml, Al⁺⁺⁺ 250 µg/ml, merthiolate 1/1000, le tout en PBS.

Comme dans le cas de l'administration orale, 3 injections sont préconisées chacune espacée de 4 semaines de la précédente.

## Revendications

1. Composition pharmaceutique destinée au traitement ou à la prévention des infections à *Helicobacter pylori* comprenant à titre de principe actif, une catalase d'*H. pylori*, sous forme purifiée.

2. Une composition selon la revendication 1, dans laquelle ladite catalase a perdu son activité enzymatique par mutation.

3. Une composition selon la revendication 1 ou 2, dans laquelle ladite catalase est encapsulée dans un vecteur de délivrance.

4. Une composition selon la revendication 3, dans laquelle le vecteur de délivrance est des liposomes.

5. Une composition selon l'une des revendications 1 à 4, qui comprend un adjuvant.

6. Une composition selon l'une des revendications 1 à 4, qui comprend un antigène d'*H. pylori* autre qu'une catalase.

7. Une composition selon la revendication 6, dans laquelle l'antigène autre que la catalase est l'uréase.

## Claims

1. Pharmaceutical composition intended for the treatment or prevention of infections by *Helicobacter pylori* comprising as its active ingredient a catalase of *H. pylori* in purified form.

2. A composition according to Claim 1 in which the said catalase has lost its enzymatic activity by mutation.

3. A composition according to Claims 1 or 2 in which the said catalase is encapsulated in an administration vehicle.

4. A composition according to Claim 3 in which the administration vehicle is liposomes.

5. A composition according to any of Claims 1 to 4 which contains an adjuvant.

6. A composition according to any of Claims 1 to 4 containing an antigen of *H. pylori* other than a catalase.

7. A composition according to Claim 6 in which the antigen other than the catalase is urease.

## Patentansprüche

1. Pharmazeutische Zusammensetzung zur Behandlung oder Vorbeugung von *Helicobacter pylori*-Infektionen, umfassend eine Katalase von *H. pylori* in gereinigter Form als Wirkstoff.

2. Zusammensetzung nach Anspruch 1, wobei die Katalase ihre Enzymaktivität durch Mutation verloren hat.

3. Zusammensetzung nach Anspruch 1 oder 2, wobei die Katalase in ein Abgabevehikel eingekapselt ist.

4. Zusammensetzung nach Anspruch 3, wobei Liposomen als Abgabevehikel dienen.

5. Zusammensetzung nach einem der Ansprüche 1 bis 4, die einen Hilfsstoff umfaßt.

6. Zusammensetzung nach einem der Ansprüche 1 bis 4, die ein anderes, von Katalase verschiedenes *H. pylori*-Antigen umfaßt.

7. Zusammensetzung nach Anspruch 6, wobei das andere, von Katalase verschiedene Antigen Urease ist.
